# EUROPEAN PATENT APPLICATION

(11) **EP 0 810 281 A2**
(43) Date of publication of application: **03.12.1997**
(21) Application number: 97303594.2
(22) Date of filing: 27.05.1997
(51) Int. Cl.: C12M 1/34

(54) **Detecting apparatus for monitoring culture broth in bio-reactor**

(30) Priority: 30.05.1996 JP 137256/96
(71) Applicant: RIKAGAKU KENKYUSHO, Wako-shi Saitama-ken (JP); Komatsugawa Chemical Engineering Co., Ltd., Tokyo (JP)
(72) Inventor: Endo, Isao, Wako-shi, Saitama-ken (JP); Nagamune, Teruyuki, Wako-shi, Saitama-ken (JP); Inoue, Kozo, Tokyo (JP); Kawamura, Kinichi, Tokyo (JP)
(74) Representative: BATCHELLOR, KIRK & CO.

(57) **Abstract**

A detecting apparatus (1) is disclosed, that comprises a plurality of measurement devices (7-11) with respective sensors (e.g. 7a, 7b) and supporting means (2) for holding the measurement devices in such a manner that the individual sensors can, when the apparatus is affixed to a bio-reactor, contact a culture broth; the turbidity, dissolved oxygen, dissolved carbon dioxide, temperature, pH, and similar or other internal environmental conditions of the culture broth being obtained by these measurement devices. Use of such a device enables the physiological activity of the organism in the culture broth to be monitored, whilst reducing the risk of ingress of foreign matter to the interior of a bio-reactor whilst simultaneously reducing disruption to the liquid flow within a bio-reactor fitted with this detecting apparatus.

## Description

The present invention relates to a detecting apparatus for monitoring a culture broth of bacteria, yeast, tissue of an animal/plant, a cell thereof, or the like cultivated in a bio-reactor.

Conventionally, bacteria, yeast, tissue of an animal/plant, a cell thereof, and so forth are cultivated within bio-reactors whose internal environments are properly controlled. The environmental conditions such as turbidity, dissolved oxygen, dissolved carbon dioxide, the temperature, pH, and so forth of the culture broth in such a bio-reactor are detected so as to monitor the physiological activity of an organism in the culture broth.

In a conventional bio-reactor, to detect the turbidity, dissolved oxygen, dissolved carbon dioxide, the temperature, pH, and so forth of the culture broth, a plurality of opening portions are formed on the side wall of the bio-reactor. Measurement devices that detect these specific environmental conditions are disposed in the bio-reactor through these opening portions.

However, with such conventional bio-reactor detectors, the opening portions for the measurement devices disposed in the bio-reactor cause foreign matter such as various bacteria and/or impurities to enter the inside of the bio-reactor, resulting in contamination of the inside of the bio-reactor which can adversely affect the culture broth. In addition, a plurality of measurement devices disposed in the bio-reactor tends to interfere with and disrupt the liquid flow, which can adversely affect cultivation of the organism e.g. the microorganisms present in the culture broth.

The present invention has been derived from a consideration of the above-described difficulties. An object of the present invention is to provide a detecting apparatus that can suppress foreign matter from entering a bio-reactor and which reduces disruption of the liquid flow therein.

The present invention provides a detecting apparatus for monitoring a culture broth in a bio-reactor, which apparatus comprises a plurality of measurement devices with respective sensors and holding means for holding said measurement devices in such a manner whereby, in use, the individual sensors can contact the culture broth.

According to the present invention, by using only one opening portion on e.g. the side wall of a bio-reactor, a group of measurement devices can be disposed therein. Thus, in addition to suppressing foreign matter from entering the inside of the bio-reactor, the detecting apparatus can also reduce disruption and disturbance to the liquid flow inside the bio-reactor. Moreover, the group of measurement devices can be easily handled and the serviceability of the detector and of the bio-reactor in which it is installed can be improved.

In embodiments of the present invention, the individual measurement devices can be compactly structured, such that output signals from each individual measurement device within the detector do not interfere with each other.

In order that the invention may be illustrated, more easily appreciated and readily carried into effect by those skilled in this art, an embodiment of a detector apparatus according to the invention will now be described purely by way of non-limiting example only, with reference to the accompanying drawings and in which:

Fig. 1 is a partially exploded view showing the principal components of such a detecting apparatus, and

Fig. 2 is a schematic diagram showing the detecting apparatus (of Fig. 1) disposed to protrude into the interior of a bio-reactor, capable of contact with a culture broth medium.

Referring to the drawings,

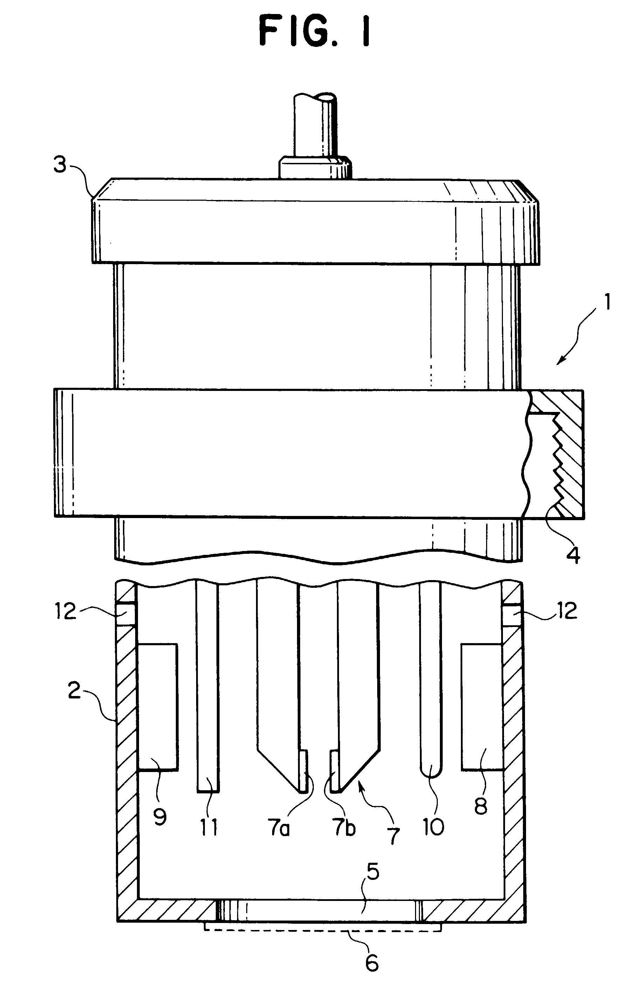

Fig. 1 is a partially exploded view showing the principal parts of a detecting apparatus according to an embodiment of the present invention. Referring to Fig. 1, the detecting apparatus 1 according to the embodiment has a hollow cylindrical housing 2. A cap 3 is threaded and serves as a top portion of the housing 2. As will be described later, a screw 4 is formed below the cap 3 for the purpose of securing the detecting apparatus 1 to the side wall of the bio-reactor. An opening portion 5 for supplying culture broth present in the bio-reactor to the inside of the housing 2 is formed at a bottom portion of the housing 2. A mesh 6 is disposed in the opening portion 5. The mesh 6 prevents or minimises bubbles from entering the inside of the housing 2.

The interior of the housing 2 is provided with a group, that is a plurality, of individual measurement devices. They are spaced apart and located approximately concentrically to the longitudinal axis of the cylinder 2.

At a central portion of the housing 2, a turbidity detector 7 is disposed. The turbidity detector 7 has a light emitting portion 7a and a light receiving portion 7b. The light emitting portion 7a emits light from, for example, a semiconductor laser diode (not shown). The light receiving portion 7b receives the light emitted from the light emitting portion 7a and guides it to a semiconductor photodiode (not shown). The turbidity of the culture broth medium present between the light emitting portion 7a and the light receiving portion 7b is detected corresponding to the transmittance of the light of the solution.

A dissolved oxygen detector 8 and a dissolved carbon dioxide detector 9 are respectively securely mounted on the inner wall of the housing 2. A temperature detector 10 is disposed between the turbidity detector 7 and the dissolved oxygen detector 8. A pH detector 11 is disposed between the turbidity detector 7 and the dissolved carbon dioxide detector 9. Signal lines (not shown) from the detectors 7 to 11 extend vertically to an upper portion of the cap 3. Such lines can be easily connected to display or monitoring devices remote from the detector housing.

A plurality of holes 12 are formed in the side wall of the housing 2 to discharge culture broth medium from the housing 2.

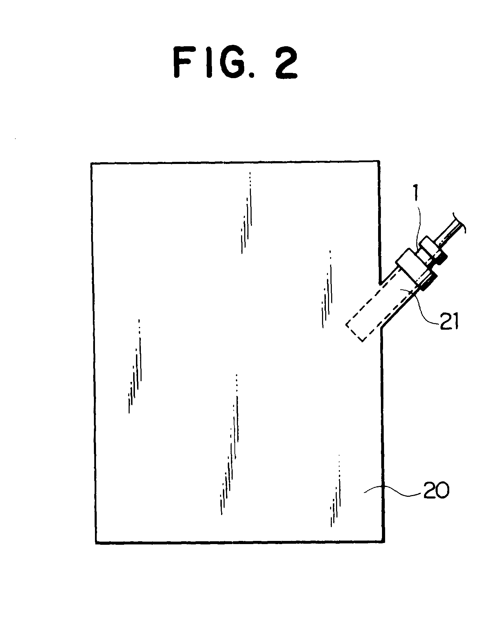

As shown in Fig. 2, the detecting apparatus 1 is formed in the shape of a vessel. The detecting apparatus 1 is disposed in a bio-reactor 20 where bacteria, yeast, tissue of an animal/plant, a cell thereof, or the like is cultivated. In other words, the detecting apparatus 1 is disposed in a cylindrical opening portion 21 that protrudes from the side wall of the bio-reactor 20 at an angle and in such a manner that at least the tip portion of the detecting apparatus 1 contacts the culture broth contained in the bio-reactor 20. The detecting apparatus 1 is secured by threading the screw 4 shown in Fig. 1 to a complementary screw (not shown) formed outside the opening portion 21.

By measuring the turbidity, dissolved oxygen, dissolved carbon dioxide, the temperature, pH, and so forth of the culture broth in the bio-reactor 20 with the detecting apparatus 1, the physiological activity of an organism in the culture broth is monitored.

As described above, in the detecting apparatus 1 according to the embodiment, since the turbidity detector 7, the dissolved oxygen detector 8, the dissolved carbon dioxide detector 9, the temperature detector 10, and the pH detector 11 are integrally structured within a single casing, they can be disposed in the bio-reactor 20 through use of only one opening portion 21.

Thus, with detecting apparatus 1 according to the embodiment, foreign matter such as various bacteria and impurities can be suppressed from entering the inside of the bio-reactor. In addition, disruption to the liquid flow in the bio-reactor 20 can be reduced. Moreover, since the individual detecting devices 7 to 11 can be easily handled (for example, they can be easily mounted and dismounted), the serviceability of the detector is enhanced.

As described above, with the detecting apparatus according to the present invention, foreign matter can be suppressed from entering the inside of the bio-reactor and the disorder of the liquid flow in the bio-reactor can be reduced in comparison with a conventional bio-reactor employing a plurality of individual detectors immersed separately in the culture broth.

Although the present invention has been shown and described with respect to a preferred embodiment thereof, it should be understood by those skilled in the art that the foregoing and various other changes, omissions, and additions in form and detail thereof may be made therein without departing from the spirit and scope of the present invention.

## Claims

1. A detecting apparatus for monitoring a culture broth in a bio-reactor, comprising:
a plurality of measurement devices with respective sensors; and
a holding means for holding said measurement devices in such a manner that the individual sensors can contact said culture broth.

2. Detecting apparatus as claimed in claim 1,
wherein said plurality of measurement devices includes at least one device selected from the group consisting of a turbidity detector, a dissolved oxygen detector, a dissolved carbon dioxide detector, a thermometer, and a pH detector.

3. Detecting apparatus as claimed in claim 1 or 2,
wherein the sensors of said measurement devices are covered with a housing, and
wherein said housing is hollow and has an opening portion.

4. Detecting apparatus as claimed in any preceding claim,
wherein the opening portion of said housing has a mesh for preventing or minimising bubbles from entering the inside of said housing.

5. Detecting apparatus as claimed in claim 3 or 4,
wherein said housing is cylindrical and includes a cap at its upper end.

6. Detecting apparatus as claimed in claim 5 further including a collar for securing the apparatus to an opening in a bio-reactor.

7. Bio-reactor apparatus fitted with detecting apparatus according to any preceding claim.

8. Use of detecting apparatus as claimed in any one of claims 1 to 6 for monitoring the internal environmental conditions of a culture broth contained in a bio-reactor.
